(19) Europäisches Patentamt
European Patent Office
Office européen des brevets

(11) **EP 3 979 903 B1**

(12) **EUROPEAN PATENT SPECIFICATION**

(45) Date of publication and mention
of the grant of the patent:
**23.07.2025 Bulletin 2025/30**

(21) Application number: **20817755.0**

(22) Date of filing: **05.06.2020**

(51) International Patent Classification (IPC):
*A61B 5/24* *(2021.01)* *A61N 1/05* *(2006.01)*
*A61N 1/36* *(2006.01)* *A61B 5/20* *(2006.01)*
*A61B 5/389* *(2021.01)* *A61B 5/392* *(2021.01)*
*A61B 5/00* *(2006.01)*

(52) Cooperative Patent Classification (CPC):
**A61N 1/0551; A61B 5/205; A61B 5/389;**
**A61B 5/392; A61B 5/4836; A61B 5/6877;**
**A61B 5/7214; A61B 5/7246; A61N 1/36139;**
A61B 2560/0214; A61B 2562/043

(86) International application number:
**PCT/AU2020/050570**

(87) International publication number:
**WO 2020/243785 (10.12.2020 Gazette 2020/50)**

(54) **METHOD AND SYSTEM FOR DETECTING NEURAL ACTIVITY**

VERFAHREN UND SYSTEM ZUR DETEKTION VON NEURONALER AKTIVITÄT

PROCÉDÉ ET SYSTÈME POUR DÉTECTER L'ACTIVITÉ NERVEUSE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **07.06.2019 AU 2019901989**

(43) Date of publication of application:
**13.04.2022 Bulletin 2022/15**

(73) Proprietor: **The Bionics Institute of Australia**
**East Melbourne, Victoria 3002 (AU)**

(72) Inventor: **FALLON, James**
**Melbourne, Victoria 3002 (AU)**

(74) Representative: **AWA Sweden AB**
**Box 45086**
**104 30 Stockholm (SE)**

(56) References cited:
**WO-A1-2019/095020     WO-A1-2019/095020**
**WO-A2-03/103484       WO-A2-2018/234825**
**US-A1- 2018 140 843**

- **AZZAM M ET AL: "Stability of egg white-**
**stabilized edible oil emulsions using**
**conductivity technique", FOOD**
**HYDROCOLLOIDS, ELSEVIER BV, NL, vol. 16,**
**no. 2, 1 March 2002 (2002-03-01), pages 105 - 110,**
**XP027304005, ISSN: 0268-005X, [retrieved on**
**20020301]**

EP 3 979 903 B1

## Description

### Technical Field

The present disclosure relates to detection of neural activity, specifically through the receiving of electrical signals from the nervous system using electrodes.

### Background

Electroceutical devices are medical devices which treat ailments using electrical impulses. Such devices may utilise bioelectric neuromodulation to treat a range of diseases or medical conditions.

One advantage of bioelectric neuromodulation devices, compared to pharmaceutical or biological treatments, is that the level of stimulation may be rapidly adjusted to respond to changing patient needs. This is known as closed-loop control. However, true closed-loop bioelectric neuromodulation requires the ability to chronically stimulate or activate neural activity, inhibit or suppress neural activity, and sense ongoing spontaneous or naturally evoked neural activity.

WO2019095020, US patent no. 11,413,452, discloses a peripheral nerve electrode array that includes a first, second and third pair of electrodes spaced from each other along a longitudinal axis of the electrode array, the second pair of electrodes being located between the first and third pairs of electrodes, and a method for treating or preventing a chronic inflammatory condition in a human subject in need thereof, comprising providing to the human subject a therapeutically effective electrical stimulation of the anterior central abdominal vagus nerve or the posterior central abdominal vagus nerve, wherein the electrical stimulation is provided through two or more previously implanted electrodes at a site below the cardiac branches and above the hepatic-celiac branches of the nerve; and whereby the chronic inflammatory condition is prevented or treated in the human subject.

WO2003103484 relates to an apparatus for measuring non-stimulated activity from a sensory nerve using spaced apart sensors. The received signals may be correlated and summed to improve the signal-to-noise ratio.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each of the appended claims.

### Summary

The invention is defined by the independent claims.

According to one aspect of the present disclosure there is provided a method of detecting neural activity in a nerve, the method comprising:

> receiving a first electrical signal from a first pair of electrodes, the first pair of electrodes comprising two first electrodes located proximate each other along the nerve;
> receiving a second electrical signal from a second pair of electrodes, the second pair of electrodes comprising two second electrodes located proximate each other along the nerve, wherein the second pair of electrodes is spaced from the first pair of electrodes along the nerve;
> applying a correlation analysis between the first and second electrical signals, including for at least one non-zero lag time, to obtain correlation data; and
> detecting, from the correlation data, at least one neural signal indicative of neural activity in the nerve, the neural signal corresponding to increased correlation between the first and second signals at the at least one non-zero lag time.

In some embodiments, the first and/or second electrical signal may have a negative signal-to-noise ratio (SNR). That is, a power of a neural signal component may be smaller than a power of a noise signal component of the first and/or second electrical signal. Conventional recording apparatus, suitable for recording evoked neural activity in response to artificial stimulation, is typically unable to record ongoing spontaneous or natural neural activity due to excessive noise in the signal. The disclosed method may provide the ability to sense and extract neural signals which would otherwise be hidden in background noise.

In some embodiments, each of the first and second pairs of electrodes may be located outside a perineurium (nerve sheath) of the nerve. Since a high signal-to noise ratio is not necessarily required, the method may detect spontaneous or natural neural activity without requiring breach or penetration of the perineurium. As such, methods according to the present disclosure may be considered minimally invasive. The electrodes being located outside the perineurium may increase the longevity of devices employing the method, and their suitability for chronic implantation.

Lag time may be understood as a time offset, conduction delay or latency between the first and second electrical signals. In some embodiments, the at least one non-zero lag time may be preselected based on a distance between the first pair of electrodes and the second pair of electrodes. Alternatively, or additionally, the at least one non-zero lag time may be preselected based on a fibre type of the nerve. The lag time may be preselected to substantially coincide with a neural signal conduction time between the first and second pairs of electrodes. For example, for a given distance between the electrode pairs, the lag time may be selected based on an anticipated conduction speed of a fibre type of interest.

An absolute value of the non-zero lag time may be selected to be greater than a threshold value. The threshold value may be set to be sufficient to distinguish signals detected at the non-zero lag time from signals detected at zero lag time. For example, the absolute value of the non-zero lag time may be above 0.1 ms, 0.2 ms, 0.3 ms or otherwise.

In some embodiments, the correlation analysis may be applied for a single non-zero lag time. In other embodiments, the correlation analysis may be applied for a plurality of non-zero lag times. The plurality of non-zero lag times may span a range of lag times. For example, the plurality of non-zero lag times may be set at increments between a maximum and minimum lag time. The plurality of non-zero lag times may include negative and positive sign lag times.

The method may further comprise categorising the neural signal as afferent or efferent based on the sign of the lag time at which the neural signal is detected. That is, the direction of travel of the neural signal in the nerve may be indicated by whether the neural signal is detected at a positive lag time or a negative lag time, depending on which pair of electrodes is first reached by the signal. For example, an neural signal may reach the first pair of electrodes before the second pair of electrodes resulting in the signal being detected at a positive lag time. The neural signal may then be categorised as afferent or efferent depending on the relative positioning of the first and second electrodes along the nerve.

Further, the method may comprise categorising a fibre type of the nerve based on a magnitude of a non-zero lag time at which the neural signal is detected. For example, for a known distance between the first and second pairs of electrodes, the non-zero lag time can be indicative of a conduction speed of the nerve. The conduction speed may then be used to categorise the nerve fibre type based on known characteristics of neural fibres.

In some embodiments, the method may also comprise applying the correlation analysis for a zero lag time to obtain the correlation data. Signals which are received at both electrodes simultaneously will generally correspond to increased correlation in the correlated data at a substantially zero lag time. The method may further comprise detecting, from the correlation data, at least one alternative signal indicative of electrical activity, the alternative signal corresponding to increased correlation between the first and second signals for a substantially zero lag time. The alternative signals may be indicative of movement or evoked neural responses to stimulation.

In some embodiments, the neural signal may correspond to one or more regions of increased correlation between the first and second signals at the at least one non-zero lag time. Similarly, in some embodiments, the alternative signal may correspond to one or more regions of increased correlation between the first and second signals at zero lag time.

In some embodiments, the one or more regions of increased correlation in the correlation data at the at least one non-zero lag time (corresponding to the neural signal) may include one or more peaks in correlation between the first and second signals, the peaks being centred at the at least one non-zero lag time. Described is also that the one or more regions of increased correlation in the correlation data at the at zero lag time (corresponding to an alternative signal) may include one or more peaks in correlation between the first and second signals, the peaks being centred at zero lag time.

In some embodiments, the nerve may be a peripheral nerve. In other embodiments, the nerve may be a central nervous system nerve. In some embodiments, the nerve may be an autonomic nervous system nerve. The ability to detect, monitor and/or record neural activity in the autonomic nervous system may be advantageous, as stimulation of autonomic nerves typically does not produce a conscious percept. In other embodiments, the nerve may be a nerve of the somatic nervous system, for example, a mixed somatosensory nerve. In some embodiments, the nerve may be myelinated. In other embodiments, the nerve may be non-myelinated.

As examples, the nerve may be the pelvic nerve, vagus nerve or sciatic nerve. However, the disclosed method is not limited to these nerves.

The ability to detect or sense neural activity, particularly ongoing spontaneous or natural neural activity may be useful for neuromodulation of peripheral nerves. In particular, the ability to detect or sense ongoing spontaneous neural activity may enable the validation of a number of potential biomarkers useful for closed-loop control of electroceutical devices. For example, the method may be useful for detection of neural activity such as afferent signalling of increasing inflammation in inflammatory bowel disease (IBD), wherein optionally therapeutic treatment is initiated or adapted in response to the detected neural activity. As IBD is a remitting/relapsing condition, there will often be periods where no therapeutic treatment is required. By monitoring afferent activity in the vagus nerve using the presently disclosed method, it may be possible to detect an increase in afferent neural activity associated with a flare (that is, an increase in inflammation) before the patient experiences symptoms of the flare. In such cases, it may be possible to initiate or increase therapeutic treatment (for example, by stimulation of the vagus nerve using an electroceutical device) in direct response to the detected increase in afferent neural activity. Continued monitoring of subsequent afferent activity may then detect a resultant decrease in afferent activity associated with a decrease in inflammation, providing an indication for cessation or reduction of the therapeutic treatment. Adaptation (e.g. initiation, cessation, increase or decrease) of therapeutic treatment in response to detected neural activity may allow for ongoing closed-loop treatment of IBD, without the patient experiencing symptoms of the disease. Such closed-loop treatment may ensure that therapeutic treatment is only applied when required or only applied to a degree that is necessary. This has potential benefits for electroceutical devices in terms of reduced power consumption and/or improved battery life and minimisation of any off-target effects or safety issues.

In other examples, the method may be useful for detection of neural activity such as bladder volume afferent signalling, for example, for closed loop control of bladder prostheses.

According to another aspect of the present disclosure, there is provided processing apparatus configured to carry out the above described method. The processing apparatus may be at least partially implantable. The processing apparatus may be wholly implantable.

The received first and second electrical signals may be amplified, filtered or otherwise processed prior to applying the correlation analysis. Accordingly, the processing apparatus may comprise a signal amplifier, signal filter and/or other types of signal processors. The processing apparatus may comprise at least two recording inputs (or channels) for receiving the first and second electrical signals. The processing apparatus may be configured to receive (and optionally record) the first and second electrical signals at a sample rate of about 10 kHz or more, for example, a sample rate of at least 10 kHz, 20 kHz, 30 kHz, 40 kHz, 50 kHz or more. The processing apparatus may be configured to amplify received signals. For example, the processing apparatus may be configured to provide at least 100 times gain to the first and/second electrical signals. The processing apparatus may be configured to provide a band pass filter, for example, at least a 10-5 kHz band pass filter.

According to another aspect of the present disclosure, there is provided a non-transitory computer-readable memory medium comprising instructions to cause a processing apparatus to perform the above described method.

According to another aspect of the present disclosure, there is provided a system for detecting neural activity in a nerve, the system comprising:

a first pair of electrodes, the first pair of electrodes comprising two first electrodes positionable proximate each other along the nerve; and
a second pair of electrodes, the second pair of electrodes comprising two second electrodes positionable proximate each other along the nerve,
wherein the second pair of electrodes is configured to be spaced from the first pair of electrodes along the nerve; and
processing apparatus configured to:

receive a first electrical signal from the first pair of electrodes;
receive a second electrical signal from the second pair of electrodes;
apply a correlation analysis between the first and second electrical signals, including for at least one non-zero lag time, to obtain correlation data; and
detect, from the correlation data, at least one neural signal indicative of neural activity in the nerve, the neural signal corresponding to increased correlation between the first and second signals at the preselected, non-zero lag time.

The provision of first and second pairs of electrodes does not preclude the provision of third, fourth, fifth or yet further electrode pairs, whether for the purposes of monitoring or applying electrical signals.

In some embodiments, at least one of the first and second electrode pairs may be comprised in an electrode mounting device adapted to mount to the nerve to electrically interface the first and second electrode pairs with the nerve. The first and second pairs of electrodes may be in a substantially fixed relationship. For example, the electrode mounting device may comprise a support which substantially maintains the relative locations and orientations of the electrodes.

In some embodiments, the electrode mounting device may comprise an electrode array, the electrode array comprising the first pair of electrodes and the second pair of electrodes. In this embodiment, the two first electrodes may be positioned proximate each other along the electrode array and the two second electrodes may be positioned proximate each other along the electrode array. The first pair of electrodes may be spaced from the second pair of electrodes along the electrode array. For example, the first and second electrode pairs may be comprised in an electrode array such as that disclosed in PCT application no. PCT/AU2018/051240 (published as WO2019095020, US patent no. 11,413,452).

The two first electrodes may be spaced from each other by a distance a1 and the two second electrodes may be spaced from each other by a distance a2. The first and second pairs of electrodes may be spaced from each other by a distance b1. The distances a1 and a2 may be substantially equal, i.e. it may be that a1=a2 or they may be different. In general, the distance b1 may be greater than the distances a1 and a2. For example, the ratio between the distance a1 or distance a2 and the distance b1 may be between 1:1.5 and 1:4, between 1:1.5 and 1:3 or about 1:2.5. In another example, the ratio may be about 1:5 or more. For example, the ratio between the distance a1 or distance a2 and the distance b1, may be about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, or more.

Alternatively, or additionally, the distance b1 may be selected based on a type, or property, of fibre of the nerve in which detection of neural activity is desired. As an example, for a known nerve fibre conduction velocity (V, e.g., 1 m/s), the distance b1 may be selected to give increased correlation (or, in some embodiments, a region and/or peak in correlation) at a specific latency (L, e.g., 2ms), for example, using the formula b1 = VL (e.g., 2 mm). The magnitude of the specific latency may be selected to be large enough that the increased correlation is adequately distinguishable from background noise present at or around 0ms, and/or selected to be small enough to minimise any signal temporal dispersion effects.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

## Brief Description of Drawings

By way of example only, embodiments of the present disclosure are now described with reference to the accompanying Figures in which:

Figure 1 shows a flowchart of steps carried out in a method of detecting a neural signal according to an embodiment of the present disclosure;

Figure 2 shows an embodiment of first and second electrode pairs for use in the method of Figure 1;

Figure 3 shows signal traces and correlation data illustrating application of the method of Figure 1 to model electrical signals;

Figure 4 shows recordings of bladder pressure (P) and corresponding first (N1) and second (N2) electrical signals from a pelvic nerve;

Figure 5 shows an output of a correlation analysis applied between the first and second electrical signals (N1 and N2) of Figure 4, together with slow afferent (SA), fast afferent (FA) and efferent (E) signal traces extracted from the correlation analysis data;

Figure 6A shows a system diagram of a system for detecting a neural signal according to an embodiment of the present disclosure;

Figure 6B shows a system diagram of a system for detecting a neural signal and applying a therapeutic treatment according to an embodiment of the present disclosure;

Figure 7 shows an electrode array according to an embodiment of the present disclosure;

Figures 8A and 8B show an electrode array according to another embodiment of the present disclosure;

Figure 9 shows a recording of bladder pressure (panel A), output of a correlation analysis between first and second electrical signals from a pelvic nerve (panel B) and afferent neural signal trace extracted from the correlation data at 1ms lag time (panel C);

Figure 10 shows a recording of bladder pressure (panel A), output of a correlation analysis between first and second electrical signals from a pelvic nerve (panel B) and efferent neural signal trace extracted from the correlation data at 1ms lag time (panel C);

Figure 11 shows an enlargement of a portion of the bladder pressure recording of Figure 10 (panel A), a corresponding portion from the correlation analysis of Figure 10 (panel B), and afferent (panel C) and efferent (panel D) neural signals extracted from the correlation data;

Figure 12 shows a recording of bladder pressure (panel A), output of a correlation analysis between first and second electrical signals from a pelvic nerve (panel B) and fast afferent and efferent neural signal traces (panels C and D) extracted from the correlation data; and

Figure 13 shows a trace representative of the change of angle in an ankle in a rat, overlaid on output of a correlation analysis between first and second electrical signals from the sciatic nerve of the rat.

**Description of Embodiments**

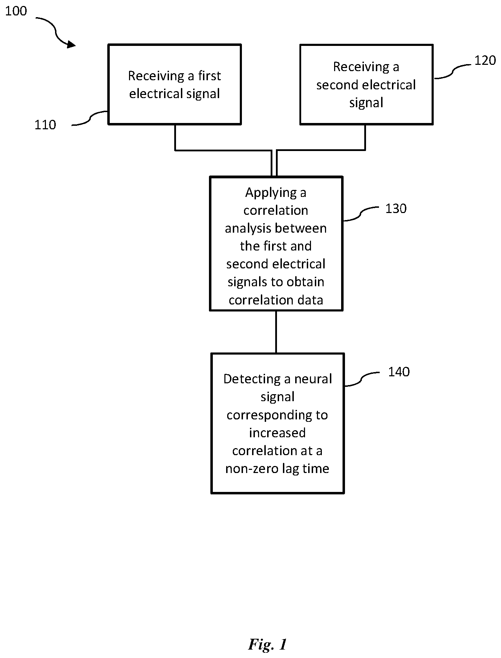

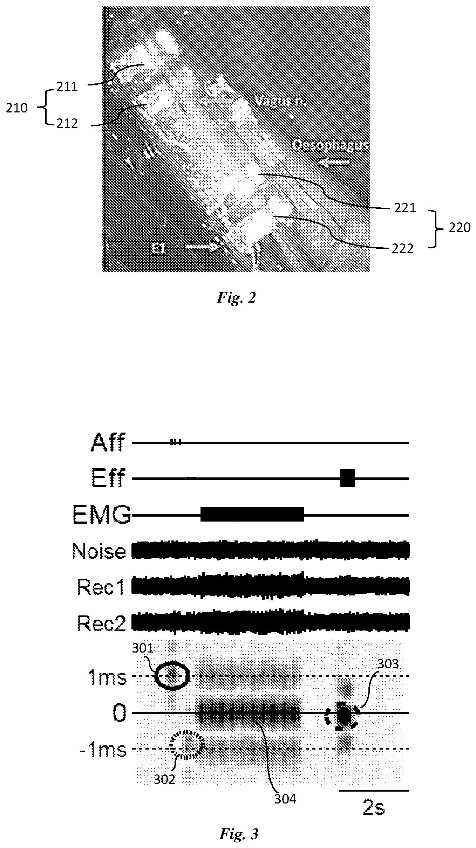

A method of detecting neural activity in a nerve according to an embodiment of the present disclosure is described with reference to flowchart 100 of Figure 1. The method comprises receiving a first electrical signal 110 and a second electrical signal 120. The received first and second electrical signals 110, 120 may be amplified, filtered or otherwise processed. The first and second electrical signals 110, 120 are received from respective first and second pairs of electrodes (for example, electrode pairs 210, 220 as shown in Figure 2). The first pair of electrodes 210 comprises two first electrodes 211, 212 located proximate each other along the nerve. Similarly, the second pair of electrodes 220 comprises two second electrodes 221, 222 located proximate each other along the nerve. As shown in Figure 2, the second pair of electrodes 220 is spaced from the first pair of electrodes 210 along the nerve. While the vagus nerve is shown in the embodiment of Figure 2, it will be appreciated that the disclosed method may be applied with respect to other nerves.

Referring again to the flowchart 100 of Figure 1, at 130, a correlation analysis is applied between the first electrical signal 110 and the second electrical signal 120 to obtain correlation data. The applying of the correlation analysis 130 is performed for one or more lag times, including for at least one non-zero lag time. The lag time may be understood as a time offset, conduction delay or latency between the first and second electrical signals, which may be a function of a distance between the first and second electrode pairs and a conduction speed of a signal.

At 140, at least one neural signal indicative of neural activity in the nerve is detected from the correlation data, the neural signal corresponding to increased correlation between the first and second electrical signals at a non-zero lag time.

Figure 3 illustrates application of the method to model signal data. Model signals traces were generated, including: 'C-fibre' afferent neural signal (Aff); slow and fast efferent neural signals (Eff); noise signal from electromyographic activity (EMG); and random background noise signal (Noise). The scale of the Aff and Eff signals is 10 times smaller than the scale of the EMG and Noise signals. Model first and second electrical signals Rec1 and Rec2 were generated by combining multiple instances of the Aff and Eff signals, and the EMG and Noise signals with appropriate delay to simulate a 1 mm spacing between electrode pairs. As can be appreciated, the EMG and large efferent activity are apparent in the model electrical signals Rec1 and Rec2. However, the small afferent and efferent neural signals of interest are swamped by the EMG and background Noise signals and are not readily detectable in either the Rec1 or Rec2 traces.

In this example, a correlation analysis was applied between the model first and second electrical signals Rec1 and Rec2 to obtain correlation data, according to the disclosed method, as shown in the lower portion of Figure 3. The correlation analysis was applied for a range of non-zero lag times (conduction delays) between approximately -2 to 2ms, and also at zero lag time. The software used for the correlation analysis was Igor Pro 8 and the main function used was 'correlate'. This function performs a linear correlation using the following formula:

$$destWaveOut[p] = \sum_{m=0}^{N-1} srcWave[m] \cdot destWaveIn[p+m]$$

The correlation data is presented graphically in the form of an activity 'heat map', in which darker areas indicate increased correlation between the first and second electrical signals and more power for a given time and conduction delay (lag time) combination. The 'heat map' was produced by repeating the correlation on blocks of the recorded signal data. The afferent neural signal (Aff), slow and fast efferent neural signals (Eff) and electromyographic signals (EMG) are each apparent in the correlation data as shown in Figure 3.

Each neural signal may appear in the graphical correlation data as a region of increased correlation between the first and second signals, indicated by a darkened band (or 'hot spot') having a central portion and flanking side portions. The central and side portions represent three peaks in correlation between the first and second electrical signals, for a given time value but corresponding to various lag times. The signal type may be categorised based on the sign of the lag time at which the band is centred. For example, referring to Figure 3, the afferent neural signal (Aff) is detected in the graphical correlation data as the dark band centred at 1ms lag time (highlighted by the solid circle 301). The slow efferent signal is detected in the graphical correlation data as the dark band centred at -1ms lag time (highlighted by the dotted circle 302). The fibre type and size may be categorised based on the magnitude of the lag time at which the band is centred. For example, the fast efferent activity is detected in the graphical correlation data as the dark band centered at a much smaller lag time of approximately -0.1 ms (highlighted by the dashed circle 303). The slow efferent signal may be distinguished from the fast efferent signal by the difference in magnitude of the lag times at which the respective signals are centred.

Signals in the graphical correlation data detected as the dark band 304 centred at substantially 0 ms (i.e. at zero lag time) are those which are received at both the first and second pair of electrodes substantially simultaneously. Such alternative signals may not be representative of signals conducting up or down the nerve fibre. For example, EMG activity (indicative of muscle activity) is substantially simultaneously recorded on both electrode pairs and appears as a dark band centred at substantially 0 ms lag time.

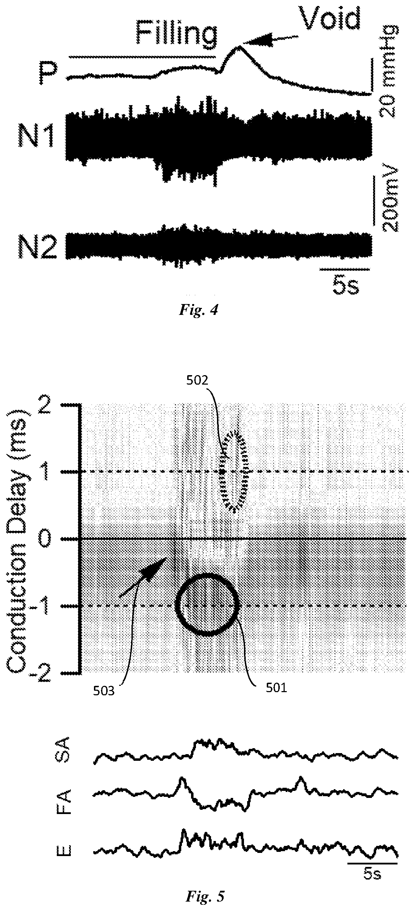

With reference to Figures 4 and 5, in another example, electrode arrays were chronically implanted on the pelvic nerve of normal adult rats, the electrode arrays each including two pairs of electrodes spaced from each other along the pelvic nerve. The rats were instrumented to allow cystometry (measurement of bladder pressure) and controlled filling of the bladder. During awake cystometry sessions, differential recording (100x gain, 10-10 kHz band pass filter; 33 kHz or 40k Hz sampling) was used to receive first and second electrical signals (N1 and N2) from the pelvic nerve, via the respective pairs of electrodes, during a spontaneous bladder voiding event.

Trace P of Figure 4 shows the bladder pressure cystometry recording over the voiding event. A gradual increase in bladder pressure can be observed, followed by a steeper rise in bladder pressure resulting from contractions of the bladder wall with an initially closed bladder sphincter and, finally, a rapid decrease in bladder pressure as the result of a bladder voiding event. The corresponding first and second electrical signal recordings from the pelvic nerve (N1 and N2) contain a signal with positive SNR during the early rise in pressure. However, the autonomic afferent and efferent neural signals of interest are not readily detectable from the recorded first and second electrical signals N1 and N2, as the signals of interest have a negative signal-to-noise ratio.

Figure 5 shows a graphical representation of correlation data obtained by applying a correlation analysis between the first and second electrical signals N1 and N2 of Figure 4. In this example, the correlation analysis was applied for a range of non-zero lag times (conduction delays), from -2 to 2 ms, and also at zero lag time. In the graph of Figure 5, darker portions indicate greater activity, or increased correlation between the first and second signals. A detected first neural signal is apparent, corresponding to the peak in correlation centred at -1 ms, indicated by the solid circle 501. The first neural signal is categorised in this particular arrangement as afferent based on the negative sign of the lag time at which the peak is centred. The absolute magnitude (1 ms) of the lag time (conduction delay) indicates that the nerve fibre type is small autonomic (based on a known distance between the electrode pairs and an inferred conduction speed of the signal). Similarly, a second peak in correlation centred at +1 ms, corresponding to a second neural signal, is indicated by the dotted ellipse 502. The second neural signal is categorised in this particular arrangement as efferent based on the positive sign of the lag time at which the peak is centred. The absolute magnitude (1 ms) of the lag time indicates that the nerve fibre type is small autonomic, based on a known distance between the electrode pairs. A third neural signal is also apparent in Figure 3, corresponding to a peak in correlation as indicated by the arrow 503. The peak indicated by the arrow 503 is centred at a negative lag time of smaller magnitude than the peaks of the first and second neural signals and, as such, can be categorised as fast afferent activity in the nerve. Individual signal traces were extracted from the correlation data and are shown beneath the heat map for each of the slow afferent (SA), fast afferent (FA) and efferent (E) signals. The signal extraction was performed by taking the appropriate row from the correlation data based on the lag time at which the relevant signal was detected.

Other embodiments may apply a correlation analysis over a narrower or wider range of lag times. Alternatively or additionally, a correlation analysis may be applied between the first and second signals for a single lag time of interest (or multiple discrete lag times of interest), for example, to isolate neural responses of one or more conduction speeds of interest.

In this example, the applying a correlation analysis between the first and second electrical signals according to the method enabled the detection of neural signals which would otherwise be hidden in background noise due to a negative signal-to-noise ratio. Further, in this example, the application of the correlation analysis for a non-zero lag time according to the method provided the ability to distinguish between and categorise the detected neural signals.

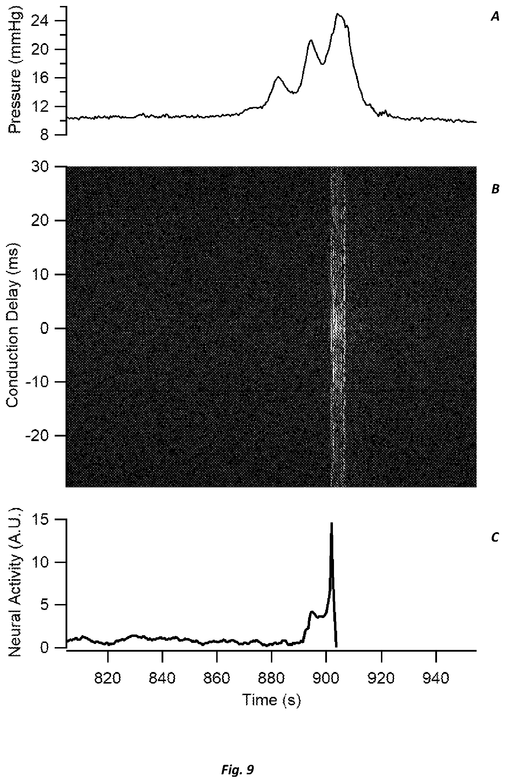

Figure 9 shows another example of data obtained using the experimental setup described above, including in which first and second electrical signal recordings are made from respective pairs of electrodes along the nerve during a bladder voiding event. Figure 9, shows the bladder pressure cystometry recording (panel A) over the voiding event, a graphical representation of the output from a correlation analysis between the two recorded signals, with areas of stronger correlation indicated in lighter shades (panel B), and an extracted trace from correlation data at 1ms conduction delay (lag time), indicative of afferent activity in the nerve (panel C). An increase in afferent activity corresponding to the second pressure increase in the bladder can be observed, before the signal is swamped by larger activity during the main pressure peak.

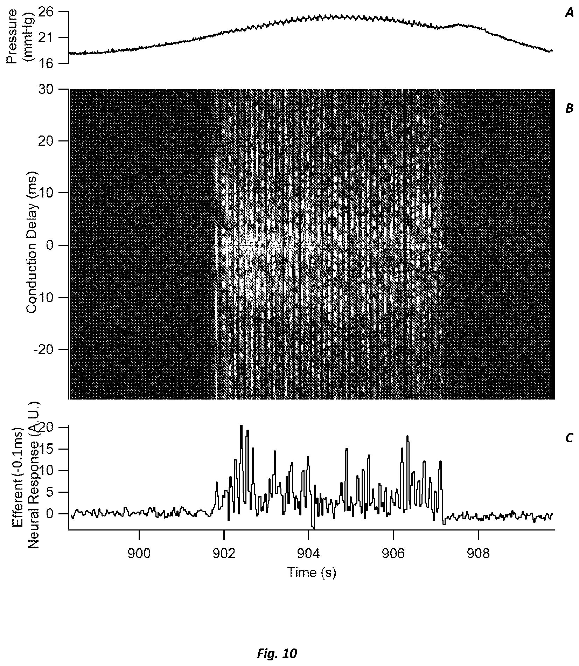

Figure 10 shows a bladder pressure cystometry trace (panel A, 1kHz sample rate), a corresponding graphical representation of correlation data (panel B, lighter colour indicates a stronger correlation) and an extracted trace from the correlation data of the 0.1ms conduction delay efferent activity signal (panel C). Periodic fluctuations are evident in the pressure trace. These fluctuations in pressure are matched by modulations in the efferent activity trace.

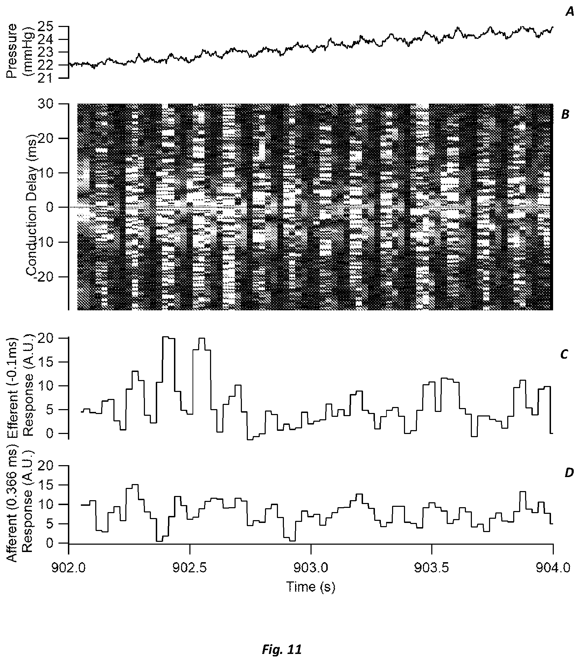

Figure 11, shows the pressure fluctuations of Figure 10 in greater detail (panel A), a corresponding detail from the correlation analysis heat map (panel B) and a trace extracted from the correlation heat map indicative of efferent activity at -0.105 ms conduction delay (panel C), and a trace extracted from the correlation heat mapindicative of afferent activity at 0.366 ms conduction delay (panel D). Both the efferent and afferent traces exhibit modulations which match the periodic pressure changes. Methods according to the present disclosure thus allow afferent and efferent neural signals to be detected simultaneously, such that any patterns or relationships between the afferent and efferent activity may be identified.

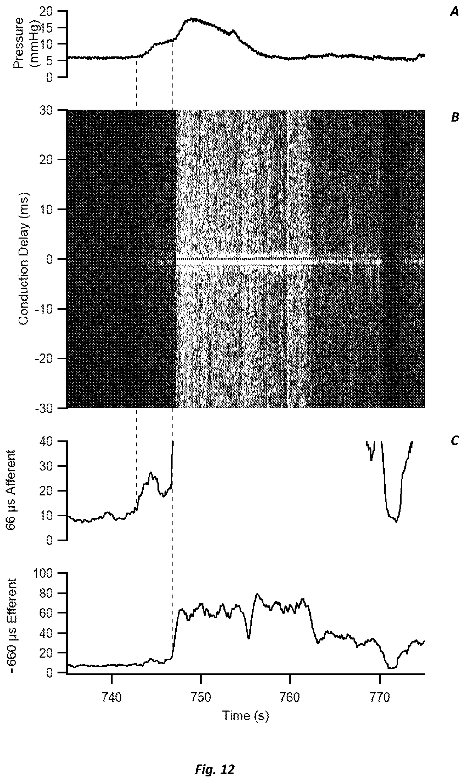

Figure 12 shows data obtained during another bladder voiding event in a rat including bladder pressure cystometry trace during the voiding event (panel A) a graphical representation of correlation data, where a lighter shade indicates a stronger correlation (panel B), and respective fast afferent and efferent signal traces extracted from the correlation data (panels C and D). From these traces, the relative timing of different neural signals during a typical bladder voiding event can be observed.

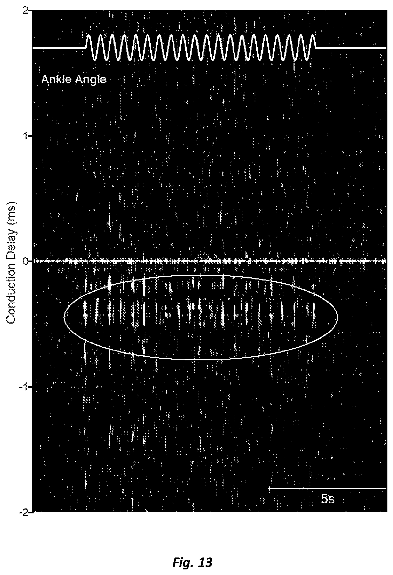

With reference to Figure 13, in another example, an electrode array was implanted on the sciatic nerve of a rat. The rat's ankle was manipulated to change the angle of the joint in a 2 Hz periodic stretching motion (white trace, top of Figure 13). A correlation analysis was performed on the signals received at the two pairs of electrodes. A graphical representation of this analysis is shown in the lower portion of Figure 13, where a lighter shade indicates a stronger correlation. As seen in the area indicated by the solid ellipse, periods of afferent neural activity were detected, corresponding in frequency to the period of the ankle stretching motion. The conduction speed of the nerve fibre was calculated at around 48mm/ms, based on the distance between the electrodes and the conduction delay (non-zero lag time), indicating that the nerve fibres conducting the detected neural signal were type A-alpha.

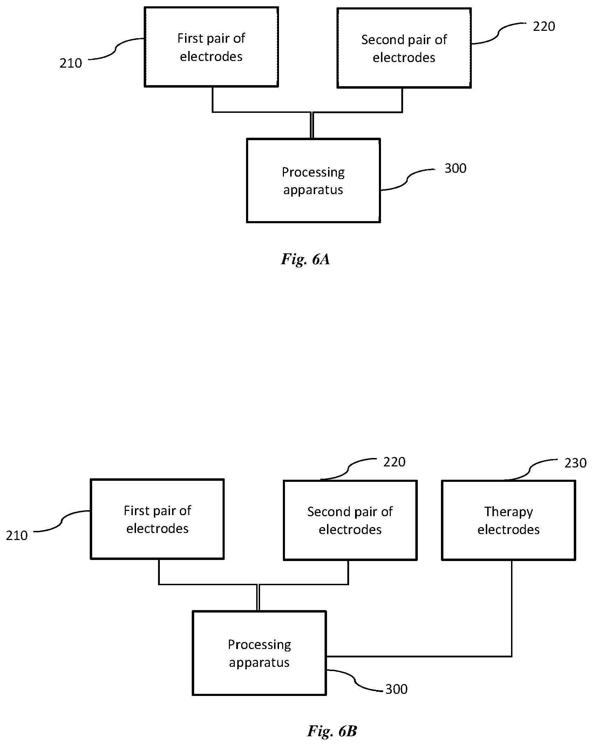

A system for detecting neural activity in a nerve according to an embodiment of the present disclosure is illustrated by system diagram 200 in Figure 6A. The system includes a first pair of electrodes 210, a second pair of electrodes 220 and processing apparatus 300.

The processing apparatus 300 may be configured to perform the method disclosed above with reference to Figure 1, for example, or otherwise.

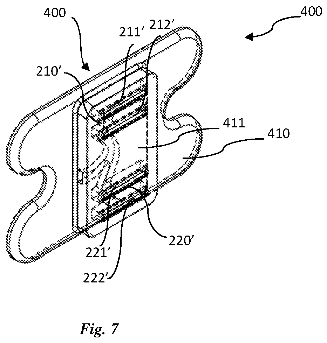

Figure 7 illustrates an electrode array 400 including first and second surface electrode pairs 210', 220' according to an embodiment of the present disclosure. The first pair of surface electrodes 210' comprises two first electrodes 211', 212' positionable proximate each other along the nerve, and the second pair of electrodes 220' comprises two second electrodes 221', 222' positionable proximate each other along the nerve. The second pair of electrodes 220' is configured to be spaced from the first pair of electrodes 210' along a nerve.

The electrode pairs 210', 220' are embedded or otherwise located in an electrode mounting device 410 of the array, which is adapted to electrically interface the first and second electrode pairs 210', 220' with the nerve. The electrode mounting device 410 comprises a support 411 that substantially maintains the relative orientation and location of the pairs of electrodes 210', 220' with respect to each other. As such, in this embodiment, the spacing between the electrodes 211', 212', 221', 222' is substantially pre-defined and fixed.

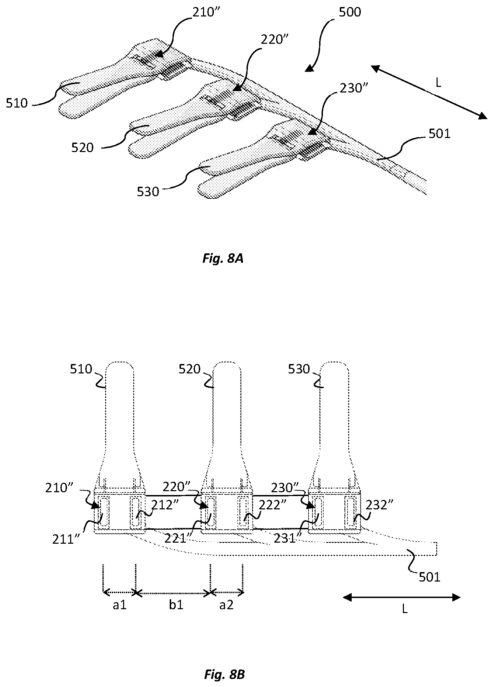

An alternative embodiment is illustrated in Figures 8A and 8B. In this embodiment, electrode array 500 includes a lead 501 that comprises electrode pairs for detecting neural activity at the nerve. The lead 501 divides into three separate branches, each branch comprising a separate electrode mounting device 510, 520, 530. Each electrode mounting device 510, 520, 530 comprises a respective pair of electrodes 210", 220", 230". In particular: the first electrode mounting device 510 comprises a first pair of electrodes 210", the first pair of electrodes comprising two first electrodes 211", 212" located proximate each other along a longitudinal direction L of the electrode array; the second electrode mounting device 520 comprises a second pair of electrodes 220", the second pair of electrodes comprising two second electrodes 221", 222" located proximate each other in the longitudinal direction L of the electrode array. In this embodiment, optionally a third electrode mounting device 530 is provided comprising a third pair of electrodes 230". The third pair of electrodes may, for example, comprise two third electrodes 231", 232" located proximate each in the longitudinal axis L of the electrode array and may be for the purposes of detecting, recording, monitoring or applying electrical signals.

It will be appreciated that other embodiments may have four, five or more pairs of electrodes provided for various purposes. Additionally, the first and second pair of electrodes need not be adjacent each other on the array, and may be separated by one or more other pairs of electrodes.

As represented in Figure 8B, the first, second and third mounting devices 510, 520, 530 are spaced from each other in the longitudinal direction L of the electrode array 500. As such, the first, second and third pairs of electrodes 210", 220" are correspondingly spaced from each other in the longitudinal direction L of the electrode array. The first electrodes 211", 212" are spaced from each other by a distance a1 and the second electrodes 221" 222" are spaced from each other by a distance a2, the distances a1 and a2 being in the longitudinal direction of the electrode array and generally from centre-to-centre of the respective electrodes. As also represented in Figure 8B, the first and second pairs of electrodes 210", 220" are spaced from each other by a distance b1, the distance b1 being in the longitudinal direction of the electrode array and generally from centre-to-centre of the closest electrodes of the adjacent pairs of electrodes. In the illustrated embodiment, the distances between the electrodes within each pair of electrodes 210", 220" is substantially the same, i.e. a1=a2. In this embodiment, the distance b1, between the first and second pairs of electrodes 210", 220" is greater than the distances a1 and a2 between the electrodes within each pair of electrodes 210", 220". The distance b1 is greater than the distance a1 and the distance a2. The ratio between the distances a1 and a2 and the distance b1 is between 1: 1.5 and 1:3, and more specifically about 1:2.5 in this embodiment. In alternative embodiments, the distances a1 and a2 between the first and second pairs of electrodes may not be equal. In some instances, such an asymmetric arrangement of electrodes may be desirable in view of anatomical and/or physiological conditions.

For example, when detecting activity in the rat pelvic nerve in the examples discussed above, the electrode pairs were spaced along the nerve with a distance b1 of approximately 2mm from each other, resulting in the slow afferent and slow efferent neural signals being detectable at lag times of approximately +/-1ms. However, in other embodiments, (e.g., when detecting signals travelling along myelinated nerve fibres) the conduction of signals between the electrode pairs may be much faster. As a result, the lag time across small distances may be very low, such that neural signals are obscured by the background noise present at and around 0ms lag time. In such embodiments, the distance b1 between the electrode pairs may be increased accordingly, thereby to increase the lag time, such that the neural signal is more clearly distinguishable from the background noise present at 0ms lag time. For example, when detecting fast afferent activity in the rat sciatic nerve (as shown in Figure 13) the electrodes pairs were spaced along the nerve at a distance b1 of approximately 19mm from each other. Conversely, in some embodiments, the distance b1 between the electrode pairs may be decreased to avoid temporal dispersion effects. The distance b1 between the electrode pairs may be selected based on an anticipated conduction speed to provide a desired lag time, while minimising temporal dispersion.

In some embodiments, detecting or sensing of neural activity, e.g. in accordance with methods and apparatus described above, particularly ongoing spontaneous or natural neural activity, may be used in conjunction with neuromodulation of peripheral nerves, e.g. as part of closed-loop control of electroceutical devices. Referring for example to Figure 6B, the apparatus may be configured generally in accordance with the apparatus described above with reference to Figure 6A, but may additionally include therapy electrodes 230 configured to apply therapeutic electrical treatment to a nerve, based on the detected neural activity. The processing apparatus 300 may therefore detect neural activity and control therapy on the basis of the detected neural therapy. In Figure 6B, while therapy electrodes 230 are illustrated as being separate from the first and second pairs of electrodes 210, 220, and may be in the form of a third pair of electrodes or otherwise, in other embodiments the first and/or second pairs of electrodes may be selectively operable as therapy electrodes.

The apparatus described with reference to Figure 6B may be useful for detection of neural activity such as afferent signalling of increasing inflammation in inflammatory bowel disease (IBD), wherein therapeutic treatment is initiated or adapted in response to the detected neural activity. As IBD is a remitting/relapsing condition, there will often be periods where no therapeutic treatment is required. By monitoring afferent activity in the vagus nerve in the present manner, it may be possible to detect an increase in afferent neural activity associated with a flare (that is, an increase in inflammation) before the patient experiences symptoms of the flare. In such cases, it may be possible to initiate or increase therapeutic treatment (for example, by stimulation of the vagus nerve using an electroceutical device) in direct response to the detected increase in afferent neural activity. Continued monitoring of subsequent afferent activity may then detect a resultant decrease in afferent activity associated with a decrease in inflammation, providing an indication for cessation or reduction of the therapeutic treatment. Adaptation (e.g. initiation, cessation, increase or decrease) of therapeutic treatment in response to detected neural activity may allow for ongoing closed-loop treatment of IBD, without the patient experiencing symptoms of the disease. Such closed-loop treatment may ensure that therapeutic treatment is only applied when required or only applied to a degree that is necessary. This has potential benefits for electroceutical devices in terms of reduced power consumption and/or improved battery life and minimisation of any off-target effects or safety issues.

In other examples, the apparatus of Figure 6B may be useful for detection of neural activity such as bladder volume afferent signalling, for example, for closed loop control of bladder prostheses.

Methods and apparatus according to embodiments of the present disclosure may use non-transitory computer-readable memory medium comprising instructions to cause processing apparatus to perform the specified steps.

In general processing apparatus used in the present disclosure may comprise one or more processors and/or data storage devices. The one or more processors may each comprise one or more processing modules and the one or more storage devices may each comprise one or more storage elements. The modules and storage elements may be at one site, e.g. in a single hand-held device, or distributed across multiple sites and interconnected by a communications network such as the internet.

The processing modules can be implemented by a computer program or program code comprising program instructions. The computer program instructions can include source code, object code, machine code or any other stored data that is operable to cause a processor to perform the methods described. The computer program can be written in any form of programming language, including compiled or interpreted languages and can be deployed in any form, including as a stand-alone program or as a module, component, subroutine or other unit suitable for use in a computing environment. The data storage device may include non-transitory computer-readable memory or otherwise.

## Claims

1. A method of detecting neural activity in a nerve, the method comprising:

   receiving a first electrical signal (110) from a first pair of electrodes, the first pair of electrodes (210) comprising two first electrodes (211, 212) located proximate each other along the nerve; and
   receiving a second electrical signal (120) from a second pair of electrodes (220), the second pair of electrodes (220) comprising two second electrodes (221, 222) located proximate each other along the nerve, wherein the second pair of electrodes (220) is spaced from the first pair of electrodes (210) along the nerve; **characterised in that** the method further comprises:

> applying a correlation analysis between the first and second electrical signals (110, 120), including for at least one non-zero lag time, to obtain correlation data; and
> detecting, from the correlation data, at least one neural signal indicative of neural activity in the nerve, the neural signal corresponding to increased correlation between the first and second signals at the at least one non-zero lag time.

2. The method of claim 1 wherein the first and/or second electrical signal (110, 120) has a negative signal-to-noise ratio.

3. The method of claim 1 or claim 2 wherein the at least one non-zero lag time is preselected based on:

> a distance between the first pair of electrodes (210) and the second pair of electrodes (220); and/or
> a fibre type of the nerve.

4. The method of any one of the preceding claims wherein the correlation analysis is applied for a plurality of non-zero lag times.

5. The method of claim 4 wherein the plurality of non-zero lag times includes negative and positive sign lag times and categorising the neural signal as afferent or efferent based on the sign of the lag time at which the neural signal is detected.

6. The method of any one of claim 4 or 5 further comprising categorising a fibre type of the nerve based on the magnitude of the lag time at which the neural signal is detected.

7. The method of any one of the preceding claims further comprising detecting, from the correlation data, at least one alternative signal indicative of electrical activity, the alternative signal corresponding to increased correlation between the first and second signals at a substantially zero lag time.

8. The method of any one of the preceding claims wherein the neural signal corresponds to one or more regions of increased correlation between the first and second signals at the at least one non-zero lag time.

9. The method of any one of the preceding claims wherein:

> each of the first and second pairs of electrodes (210, 220) are located outside a perineurium of the nerve; or
> wherein the nerve is a peripheral nerve; or
> wherein the nerve is an autonomic nervous system nerve; or
> wherein the nerve is myelinated; or
> wherein the nerve is non-myelinated.

10. Processing apparatus (300) configured to carry out the method of any one of the preceding claims.

11. A non-transitory computer-readable memory medium comprising instructions to cause a processing apparatus to perform the method of any one of claims 1 to 9.

12. A system (200) for detecting neural activity in a nerve, the system (200) comprising:

> a first pair of electrodes (210), the first pair of electrodes (210) comprising two first electrodes (211, 212) positionable proximate each other along the nerve; and
> a second pair of electrodes (220), the second pair of electrodes (220) comprising two second electrodes (221, 222) positionable proximate each other along the nerve,
> wherein the second pair of electrodes (220) is configured to be spaced from the first pair of electrodes (210) along the nerve; and
> processing apparatus (300) configured to:
>
> > receive a first electrical signal (110) from the first pair of electrodes; and

receive a second electrical signal (120) from the second pair of electrodes;

**characterised in that** the processing apparatus (300) is further configured to:

apply a correlation analysis between the first and second electrical signals, including for at least one non-zero lag time, to obtain correlation data; and
detect, from the correlation data, at least one neural signal indicative of neural activity in the nerve, the neural signal corresponding to increased correlation between the first and second signals at the preselected, non-zero lag time.

13. The system (200) of claim 12 wherein at least one of the first and second electrode pairs (210, 220) is comprised in an electrode mounting device (410), the electrode mounting device (410) adapted to mount to the nerve to electrically interface the first and second electrode pairs (210, 220) with the nerve.

14. The system (200) of claim 13 wherein the electrode mounting device (410) comprises an electrode array (500), the electrode array (500) comprising the first pair of electrodes (210) and the second pair of electrodes (220). wherein:

the two first electrodes (211, 212) are positioned proximate each other along the electrode array (500);
the two second electrodes (221, 222) are positioned proximate each other along the electrode array; and
the first pair of electrodes is spaced from the second pair of electrodes along the electrode array (500).

15. The system (200) of any one of claims 12 to 14, wherein the neural signal corresponds to one or more regions of increased correlation between the first and second signals at the at least one non-zero lag time.


**Patentansprüche**

1. Verfahren zum Detektieren neuronaler Aktivität in einem Nerv, wobei das Verfahren umfasst:

Empfangen eines ersten elektrischen Signals (110) von einem ersten Paar von Elektroden, wobei das erste Paar von Elektroden (210) zwei erste Elektroden (211, 212) umfasst, die nahe beieinander entlang des Nervs angeordnet sind; und
Empfangen eines zweiten elektrischen Signals (120) von einem zweiten Paar von Elektroden (220), wobei das zweite Paar von Elektroden (220) zwei zweite Elektroden (221, 222) umfasst, die nahe beieinander entlang des Nervs angeordnet sind, wobei das zweite Paar von Elektroden (220) von dem ersten Paar von Elektroden (210) entlang des Nervs beabstandet ist;
**dadurch gekennzeichnet, dass** das Verfahren ferner umfasst:

Anwenden einer Korrelationsanalyse zwischen dem ersten und dem zweiten elektrischen Signal (110, 120), einschließlich mindestens einer Verzögerungszeit ungleich Null, um Korrelationsdaten zu erhalten; und
Detektieren mindestens eines neuronalen Signals, das neuronale Aktivität in dem Nerv angibt, aus den Korrelationsdaten, wobei das neuronale Signal einer erhöhten Korrelation zwischen dem ersten und dem zweiten Signal bei der mindestens einen Verzögerungszeit ungleich Null entspricht.

2. Verfahren nach Anspruch 1, wobei das erste und/oder zweite elektrische Signal (110, 120) ein negatives Signal-Rausch-Verhältnis aufweist.

3. Verfahren nach Anspruch 1 oder Anspruch 2, wobei die mindestens eine Verzögerungszeit ungleich Null vorgewählt wird basierend auf:
einem Abstand zwischen dem ersten Paar von Elektroden (210) und dem zweiten Paar von Elektroden (220); und/oder einem Fasertyp des Nervs.

4. Verfahren nach einem der vorhergehenden Ansprüche, wobei die Korrelationsanalyse auf eine Vielzahl von Verzögerungszeiten ungleich Null angewendet wird.

5. Verfahren nach Anspruch 4, wobei die Vielzahl von Verzögerungszeiten ungleich Null Verzögerungszeiten mit negativem und positivem Vorzeichen einschließt, und das neuronale Signal als afferent oder efferent basierend auf dem Vorzeichen der Verzögerungszeit, zu der das neuronale Signal detektiert wird, kategorisiert wird.

6. Verfahren nach einem der Ansprüche 4 oder 5, ferner umfassend Kategorisieren eines Fasertyps des Nervs basierend auf dem Betrag der Verzögerungszeit, zu der das neuronale Signal detektiert wird.

7. Verfahren nach einem der vorhergehenden Ansprüche, ferner umfassend Detektieren mindestens eines alternativen Signals, das elektrische Aktivität angibt, aus den Korrelationsdaten, wobei das alternative Signal einer erhöhten Korrelation zwischen dem ersten und dem zweiten Signal bei einer Verzögerungszeit von im Wesentlichen Null entspricht.

8. Verfahren nach einem der vorhergehenden Ansprüche, wobei das neuronale Signal einer oder mehreren Regionen erhöhter Korrelation zwischen dem ersten und dem zweiten Signal bei der mindestens einen Verzögerungszeit ungleich Null entspricht.

9. Verfahren nach einem der vorhergehenden Ansprüche, wobei:

jedes der ersten und zweiten Paare von Elektroden (210, 220) sich außerhalb eines Perineuriums des Nervs befindet; oder
wobei der Nerv ein peripherer Nerv ist; oder
wobei der Nerv ein Nerv des autonomen Nervensystems ist; oder
wobei der Nerv myelinisiert ist; oder
wobei der Nerv nicht-myelinisiert ist.

10. Verarbeitungsvorrichtung (300), die dazu ausgelegt ist, das Verfahren nach einem der vorhergehenden Ansprüche auszuführen.

11. Nichtflüchtiges computerlesbares Speichermedium, das Anweisungen umfasst, um zu bewirken, dass eine Verarbeitungsvorrichtung das Verfahren nach einem der Ansprüche 1 bis 9 durchführt.

12. System (200) zum Detektieren neuronaler Aktivität in einem Nerv, wobei das System (200) umfasst:

ein erstes Paar von Elektroden (210), wobei das erste Paar von Elektroden (210) zwei erste Elektroden (211, 212) umfasst, die nahe beieinander entlang des Nervs positionierbar sind; und
ein zweites Paar von Elektroden (220), wobei das zweite Paar von Elektroden (220) zwei zweite Elektroden (221, 222) umfasst, die nahe beieinander entlang des Nervs positionierbar sind,
wobei das zweite Paar von Elektroden (220) dazu ausgelegt ist, von dem ersten Paar von Elektroden (210) entlang des Nervs beabstandet zu sein; und
eine Verarbeitungsvorrichtung (300), die ausgelegt ist zum:

Empfangen eines ersten elektrischen Signals (110) von dem ersten Paar von Elektroden; und
Empfangen eines zweiten elektrischen Signals (120) von dem zweiten Paar von Elektroden;
**dadurch gekennzeichnet, dass** die Verarbeitungsvorrichtung (300) ferner ausgelegt ist zum: Anwenden einer Korrelationsanalyse zwischen dem ersten und dem zweiten elektrischen Signal, einschließlich mindestens einer Verzögerungszeit ungleich Null, um Korrelationsdaten zu erhalten; und
Detektieren mindestens eines neuronalen Signals, das neuronale Aktivität in dem Nerv angibt, aus den Korrelationsdaten, wobei das neuronale Signal erhöhter Korrelation zwischen dem ersten und dem zweiten Signal zu der vorgewählten Verzögerungszeit ungleich Null entspricht.

13. System (200) nach Anspruch 12, wobei mindestens eines von dem ersten und dem zweiten Elektrodenpaar (210, 220) in einer Elektrodenmontageeinrichtung (410) vorliegt, wobei die Elektrodenmontageeinrichtung (410) dazu eingerichtet ist, an dem Nerv montiert zu werden, um das erste und das zweite Elektrodenpaar (210, 220) elektrisch mit dem Nerv zu koppeln.

14. System (200) nach Anspruch 13, wobei die Elektrodenmontageeinrichtung (410) ein Elektrodenarray (500) umfasst, wobei das Elektrodenarray (500) das erste Paar von Elektroden (210) und das zweite Paar von Elektroden (220) umfasst.
wobei:

die zwei ersten Elektroden (211, 212) entlang des Elektrodenarrays (500) nahe beieinander positioniert sind;
die zwei zweiten Elektroden (221, 222) entlang des Elektrodenarrays nahe beieinander positioniert; und
das erste Paar von Elektroden von dem zweiten Paar von Elektroden entlang des Elektrodenarrays (500) beabstandet ist.

15. System (200) nach einem der Ansprüche 12 bis 14, wobei das neuronale Signal einer oder mehreren Regionen mit erhöhter Korrelation zwischen dem ersten und dem zweiten Signal bei der mindestens einen Verzögerungszeit ungleich Null entspricht.

**Revendications**

1. Procédé de détection de l'activité nerveuse dans un nerf, le procédé comprenant :

   la réception d'un premier signal électrique (110) provenant d'une première paire d'électrodes, la première paire d'électrodes (210) comprenant deux premières électrodes (211, 212) situées à proximité l'une de l'autre le long du nerf ; et
   la réception d'un second signal électrique (120) provenant d'une seconde paire d'électrodes (220), la seconde paire d'électrodes (220) comprenant deux secondes électrodes (221, 222) situées à proximité l'une de l'autre le long du nerf, la seconde paire d'électrodes (220) étant espacée de la première paire d'électrodes (210) le long du nerf ;
   **caractérisé en ce que** le procédé comprend en outre :

   l'application d'une analyse de corrélation entre les premier et second signaux électriques (110, 120), comprenant pour au moins un temps de latence non nul, afin d'obtenir des données de corrélation ; et
   la détection, à partir des données de corrélation, d'au moins un signal neuronal indiquant une activité nerveuse dans le nerf, le signal neuronal correspondant à une corrélation accrue entre les premier et second signaux pendant l'au moins un temps de latence non nul.

2. Procédé selon la revendication 1 le premier et/ou le second signal électrique (110, 120) ayant un rapport signal/bruit négatif.

3. Procédé selon la revendication 1 ou selon la revendication 2 l'au moins un temps de latence non nul étant présélectionné sur la base de :

   une distance entre la première paire d'électrodes (210) et la seconde paire d'électrodes (220) ; et/ou
   un type de fibre du nerf.

4. Procédé selon l'une quelconque des revendications précédentes, l'analyse de corrélation étant appliquée pour une pluralité de temps de latence non nuls.

5. Procédé selon la revendication 4, la pluralité de temps de latence non nuls comprenant des temps de latence de signe négatif et positif et la catégorisation du signal neuronal comme afférent ou efférent sur la base du signe du temps de latence auquel le signal neuronal est détecté.

6. Procédé selon l'une quelconque des revendications 4 ou 5, comprenant en outre la catégorisation d'un type de fibre du nerf sur la base de l'ampleur du temps de latence auquel le signal neuronal est détecté.

7. Procédé selon l'une quelconque des revendications précédentes, comprenant en outre la détection, à partir des données de corrélation, d'au moins un signal alternatif indicatif d'une activité électrique, le signal alternatif correspondant à une corrélation accrue entre les premier et second signaux pendant un temps de latence sensiblement nul.

8. Procédé selon l'une quelconque des revendications précédentes, le signal neuronal correspondant à une ou plusieurs régions de corrélation accrue entre les premier et second signaux pendant l'au moins un temps de latence non nul.

9. Procédé selon l'une quelconque des revendications précédentes,

chacune des première et seconde paires d'électrodes (210, 220) étant située à l'extérieur d'un périnèvre du nerf ; ou

le nerf étant un nerf périphérique ; ou

le nerf étant un nerf du système nerveux autonome ; ou le nerf étant myélinisé ; ou

le nerf étant non myélinisé.

10. Appareil de traitement (300) configuré pour mettre en œuvre le procédé selon l'une quelconque des revendications précédentes.

11. Support de mémoire non transitoire lisible par ordinateur comprenant des instructions pour amener un appareil de traitement à réaliser le procédé selon l'une quelconque des revendications 1 à 9.

12. Système (200) de détection de l'activité nerveuse dans un nerf, le système (200) comprenant :

une première paire d'électrodes (210), la première paire d'électrodes (210) comprenant deux premières électrodes (211, 212) positionnables à proximité l'une de l'autre le long du nerf ; et

une seconde paire d'électrodes (220), la seconde paire d'électrodes (220) comprenant deux secondes électrodes (221, 222) positionnables à proximité l'une de l'autre le long du nerf,

la seconde paire d'électrodes (220) étant configurée pour être espacée de la première paire d'électrodes (210) le long du nerf ; et

un appareil de traitement (300) configuré pour :

recevoir un premier signal électrique (110) provenant de la première paire d'électrodes ; et

recevoir un second signal électrique (120) provenant de la seconde paire d'électrodes ;

**caractérisé en ce que** l'appareil de traitement (300) est en outre configuré pour :

appliquer une analyse de corrélation entre les premier et second signaux électriques, comprenant pour au moins un temps de latence non nul, afin d'obtenir des données de corrélation ; et

détecter, à partir des données de corrélation, au moins un signal neuronal indiquant une activité nerveuse dans le nerf, le signal neuronal correspondant à une corrélation accrue entre les premier et second signaux pendant le temps de latence non nul présélectionné.

13. Système (200) selon la revendication 12, au moins une des premières et secondes paires d'électrodes (210, 220) étant comprise dans un dispositif de montage d'électrodes (410), le dispositif de montage d'électrodes (410) étant adapté pour se monter sur le nerf afin d'interfacer électriquement les premières et secondes paires d'électrodes (210, 220) avec le nerf.

14. Système (200) selon la revendication 13, le dispositif de montage d'électrodes (410) comprenant un réseau d'électrodes (500), le réseau d'électrodes (500) comprenant la première paire d'électrodes (210) et la seconde paire d'électrodes (220).
dans lequel :

les deux premières électrodes (211, 212) étant positionnées à proximité l'une de l'autre le long du réseau d'électrodes (500) ;

les deux secondes électrodes (221, 222) étant positionnées à proximité l'une de l'autre le long du réseau d'électrodes ; et

la première paire d'électrodes étant espacée de la seconde paire d'électrodes le long du réseau d'électrodes (500).

15. Système (200) selon l'une quelconque des revendications 12 à 14, le signal neuronal correspondant à une ou plusieurs régions de corrélation accrue entre les premier et second signaux pendant l'au moins un temps de latence non nul.

100

Receiving a first
electrical signal

110

Receiving a
second electrical
signal

120

Applying a
correlation
analysis between
the first and
second electrical
signals to obtain
correlation data

130

Detecting a neural
signal
corresponding to
increased
correlation at a
non-zero lag time

140

*Fig. 1*

Fig. 2

Fig. 3

*Fig. 4*

*Fig. 5*

First pair of electrodes — 210

Second pair of electrodes — 220

Processing apparatus — 300

**Fig. 6A**

First pair of electrodes — 210

Second pair of electrodes — 220

Therapy electrodes — 230

Processing apparatus — 300

**Fig. 6B**

*Fig. 7*

*Fig. 8A*

*Fig. 8B*

**Fig. 9**

*Fig. 10*

Fig. 11

*Fig. 12*

*Fig. 13*

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019095020 A
- US 11413452 B

- WO 2003103484 A
- AU 2018051240 W